(19) Europäisches Patentamt
European Patent Office
Office européen des brevets

(11) Veröffentlichungsnummer: **0 072 528**
**B1**

# (12) EUROPÄISCHE PATENTSCHRIFT

(45) Veröffentlichungstag der Patentschrift: **14.05.86**

(51) Int. Cl.[4]: **C 07 C 127/00,** C 07 C 157/00, A 01 N 47/28, C 07 C 87/28, C 07 C 93/14, C 07 C 149/42, C 07 C 119/10

(21) Anmeldenummer: **82107261.8**

(22) Anmeldetag: **11.08.82**

## (54) (Thio-) Harnstoffe, ein Verfahren zu ihrer Herstellung und ihre Verwendung als Pflanzenschutzmittel.

(30) Priorität: **19.08.81 DE 3132690**

(43) Veröffentlichungstag der Anmeldung: **23.02.83 Patentblatt 83/8**

(45) Bekanntmachung des Hinweises auf die Patenterteilung: **14.05.86 Patentblatt 86/20**

(84) Benannte Vertragsstaaten: **BE CH DE FR GB IT LI NL**

(56) Entgegenhaltungen:
**EP - A - 0 000 019**
**EP - A - 0 000 376**
**DE - A - 1 518 688**
**DE - A - 2 210 603**
**DE - A - 2 543 888**
**DE - A - 2 732 257**
**FR - A - 2 439 772**
**US - A - 3 701 807**
**US - A - 3 761 241**

**CHEMICAL ABSTRACTS, Band 91, Nr. 1, 2. Juli 1979, Seite 149, Nr. 1357n, Columbus, Ohio, USA**
**CHEMICAL ABSTRACTS, Band 91, Nr. 1, 2. Juli 1979, Seite 149, Nr. 1358p, Columbus, Ohio, USA**
**CHEMICAL ABSTRACTS, Band 94, Nr. 15, 13. April 1981, Seite 202, Nr. 116002x, Columbus, Ohio, USA**

(73) Patentinhaber: **BAYER AG, Konzernverwaltung RP Patentabteilung, D-5090 Leverkusen 1 Bayerwerk (DE)**

(72) Erfinder: **Grohe, Klaus, Dr., Am Wasserturm 10, D-5068 Odenthal (DE)**
Erfinder: **Paul, Volker, Dr., Ahornstrasse 5, D-5650 Solingen (DE)**

(56) Entgegenhaltungen: (Fortsetzung)
**CHEMICAL ABSTRACTS, Band 94, Nr. 15, 13. April 1981, Seite 202, Nr. 116003y, Columbus, Ohio, USA**
**TETRAHEDRON LETTERS, Nr. 29, 1973, Seiten 2771-2774, Pergamon Press, GB. F. DUDRAGNE et al.: "Synthèse de N-fluoramines par CF3OF. Application aux bases de Schiff"**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue (Thio-) Harnstoffe, ein Verfahren zu ihrer Herstellung, Zwischenprodukte dafür und ihre Verwendung als Pflanzenschutzmittel.

Seit längerem werden Schwermetallsalze der Ethylen-bis-dithiocarbaminsäure, insbesondere deren Zinksalze im Pflanzenschutz zur Bekämpfung von phytopathogenen Pilzen, eingesetzt (vgl. R. Wegler, Chemie der Pflanzenschutz- und Schädlingsbekämpfungsmittel, Bd. 2, 565, Springer-Verlag, Berlin, Heidelberg, New York 1970). Deren Wirkung ist jedoch bei niedrigen Aufwandmengen und -konzentrationen nicht immer ganz befriedigend.

Weiterhin sind (Thio-)Harnstoffe, wie z.B. der N-(4-Chlorbenzyl)-N-cyclopentyl-N'-phenylharnstoff,
mit fungizider Wirkung bekannt (vgl. DE-A 2 732 257).

Es wurden neue (Thio-)Harnstoffe der allgemeinen Formel (I)

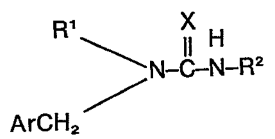

(I)

in welcher

$R^1$ für Cyclopentyl oder 2,2,2-Trifluorethyl steht,

$R^2$ für Methyl, Cyclohexyl oder für gegebenenfalls durch 4-Methyl, 4-Trifluormethoxy, 4-Trifluormethylthio, 2-Trifluormethylthio, 4-Trifluormethylthio-3-chlor, 3-Chlor-4-fluor, 2-Chlor-2-fluor-1,1-difluorethoxy oder 3,4 Dichlor substituiertes Phenyl steht,

Ar für gegebenenfalls durch 4-Chlor, 2-Trifluormethyl, 4-Trifluormethyl, 4-Trifluormethoxy, 4-Trifluormethylthio, 2-Fluor oder 3-Fluor substituiertes Phenyl steht, wobei mindestens einer der Reste $R^1$, $R^2$ und Ar Fluor oder einen fluorhaltigen Substituenten trägt und

X für Sauerstoff oder Schwefel steht,
gefunden.

Weiterhin wurde gefunden, dass man die (Thio-)Harnstoffe der Formel (I)

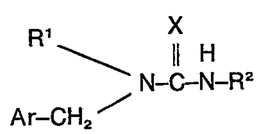

(I),

in welcher

$R^1$ für Cyclopentyl oder 2,2,2-Trifluorethyl steht,

$R^2$ für Methyl, Cyclohexyl oder für gegebenenfalls durch 4-Methyl, 4-Trifluormethoxy, 4-Trifluormethylthio, 2-Trifluormethylthio, 4-Trifluormethylthio-3-chlor, 3-Chlor-4-fluor, 2-Chlor-2-fluor-1,1-difluorethoxy oder 3,4 Dichlor substituiertes Phenyl steht,

Ar für gegebenenfalls durch 4-Chlor, 2-Trifluormethyl, 4-Trifluormethyl, 4-Trifluormethoxy, 4-Trifluormethylthio, 2-Fluor oder 3-Fluor substituiertes Phenyl steht, wobei mindestens einer der Reste $R^1$, $R^2$ und Ar Fluor oder einen fluorhaltigen Substituenten trägt und

X für Sauerstoff oder Schwefel steht,
erhält, wenn man sekundäre Amine der allgemeinen Formel (II)

$$ArCH_2-\overset{H}{N}-R^1 \quad (II),$$

in welcher

Ar und $R^1$ die bei Formel (I) angegebene Bedeutung besitzen,
mit (Thio-)Isocyanaten der Formel (III)

$$R^2-NCX \quad (III),$$

in welcher

$R^2$ und X die bei Formel (I) angegebene Bedeutung besitzen,
gegebenenfalls in einem Verdünnungsmittel bei einer Temperatur von −20 bis 150 °C umsetzt.

Die für die Herstellung der neuen (Thio-)Harnstoffe der Formel (I) benötigten Amine der Formel (II) sind neu. Sie können hergestellt werden, wenn man Aldimine der Formel (IV)

$$Ar-CH=N-R^1 \quad (IV),$$

in welcher

Ar und $R^1$ die bei Formel (I) angegebene Bedeutung besitzen,
in einem Verdünnungsmittel mit einem Hydrierungsmittel umsetzt.

Die Aldimine der Formel (IV) sind neu. Sie können hergestellt werden, wenn man Aldehyde der Formel (V)

$$ArCHO \quad (V),$$

in welcher

Ar die bei Formel (I) angegebene Bedeutung besitzt,
mit primären Aminen der Formel (VI)

$$R^1-NH_2 \quad (VI),$$

in welcher

$R^1$ die bei Formel (I) angegebene Bedeutung besitzt,
gegebenenfalls in einem Verdünnungsmittel bei einer Temperatur von 0 bis 100 °C umsetzt.

Die neuen (Thio-)Harnstoffe der Formel (I) weisen starke fungizide Eigenschaften auf. Sie zeigen gegenüber dem Stand der Technik, insbesondere bei niedrigen Aufwandmengen und -konzentrationen, eine bessere Wirksamkeit.

Neben den in den Herstellungsbeispielen beschriebenen Verbindungen der Formel (I) seien im einzelnen die folgenden Verbindungen genannt:

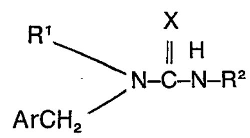

| R¹ | R² | Ar | X |
|---|---|---|---|
| 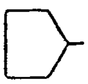 | 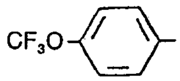 | 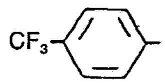 | O |
| 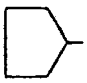 | 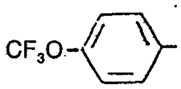 | 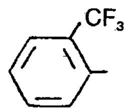 | O |
| 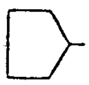 | 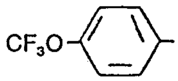 | 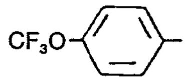 | O |
| 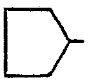 | 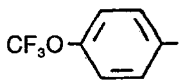 | 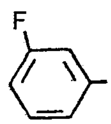 | O |
| 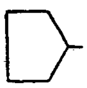 | 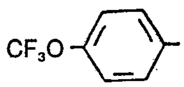 | 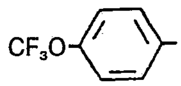 | O |
| 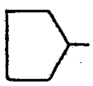 | 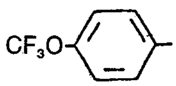 | 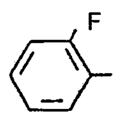 | O |
| 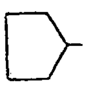 | 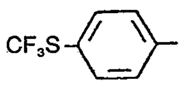 | 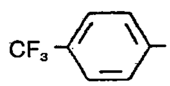 | O |
| 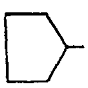 | 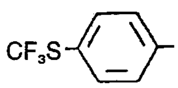 | 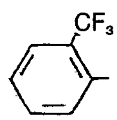 | O |
| 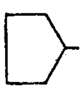 | 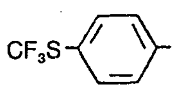 | 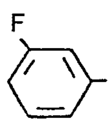 | O |
| 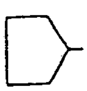 | 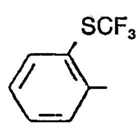 | 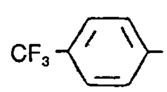 | O |
| 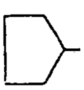 | 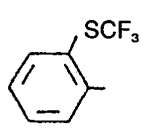 | 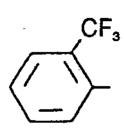 | O |
| 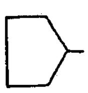 | 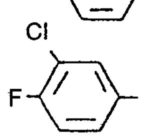 | 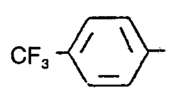 | O |
| 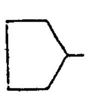 | 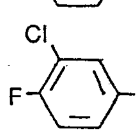 | 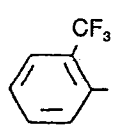 | O |

Verwendet man beispielsweise N-3-Fluorbenzyl-N-cyclopentylamin und Phenylisocyanat als Ausgangsstoffe, so kann der Reaktionsablauf durch das folgende Formelschema wiedergegeben werden

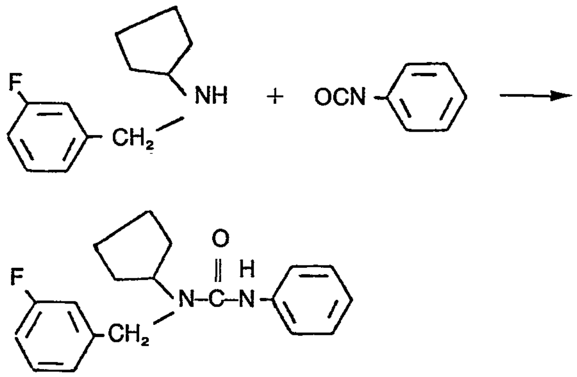

Die bei der Durchführung der erfindungsgemässen Umsetzung eingesetzten sekundären Amine und (Thio-)Isocyanate sind durch Formel (II) und Formel (III) allgemein definiert. Bei diesen Formeln haben die Reste Ar, $R^2$ und $R^1$ die Bedeutungen, die bereits im Zusammenhang mit der Beschreibung der erfindungsgemässen Stoffe der Formel (I) vorzugsweise und besonders bevorzugt für diese Reste genannt wurden.

Die im erfindungsgemässen Verfahren eingesetzten sekundären Amine der Formel (II) sind neu. Ihre Herstellung wird weiter unten beschrieben. Die eingesetzten Isocyanate der Formel (III) sind allgemein bekannte Verbindungen.

Die erfindungsgemässe Umsetzung der sekundären Amine der Formel (II) mit den (Thio-)Isocyanaten der Formel (III) kann in einem Verdünnungsmittel durchgeführt werden.

In besonderen Fällen, beispielsweise wenn die Reaktionskomponenten bei der Umsetzungstemperatur flüssig sind, kann ohne Verdünnungsmittel umgesetzt werden. Bevorzugt wird in einem Verdünnungsmittel umgesetzt.

Als Verdünnungsmittel können inerte organische Lösungsmittel, beispielsweise Ether wie Dioxan, Kohlenwasserstoffe wie Cyclohexan, aromatische Kohlenwasserstoffe wie Benzol und Toluol, chlorierte Kohlenwasserstoffe wie Chloroform und Nitrile wie Acetonitril verwendet werden.

Pro Mol sekundäres Amin der Formel (II) werden etwa 100 bis 500 ml Verdünnungsmittel eingesetzt.

Die erfindungsgemässe Umsetzung wird bei einer Temperatur von −20 bis 150 °C, vorzugsweise 0 bis 100 °C ausgeführt.

In besonderen Fällen, beispielsweise wenn die Umsetzungstemperatur oberhalb des Siedepunktes des eingesetzten Lösungsmittels liegt, kann unter erhöhtem Druck bis etwa 20 bar umgesetzt werden. Vorzugsweise wird bei Normaldruck umgesetzt.

Die erfindungsgemässe Umsetzung kann beispielsweise wie folgt durchgeführt werden:

Im Verdünnungsmittel wird das sekundäre Amin vorgelegt. Dazu wird unter Eiskühlung das Isocyanat gegeben. Anschliessend wird etwa ½ Stunde bei Raumtemperatur und anschliessend etwa ½ Stunde bei leicht erhöhter Temperatur von 30 bis 60 °C gerührt. Nach Abdestillieren des Verdünnungsmittels können die erhaltenen erfindungsgemässen Harnstoffe durch Kristallisation gereinigt werden.

Die im erfindungsgemässen Verfahren eingesetzten sekundären Amine der Formel (II) sind neu. Sie werden durch Hydrierung von Aldiminen der Formel (IV) hergestellt.

Verwendet man beispielsweise 3-Fluorbenzaldehyd-cyclopentylimin und Natriumborhydrid als Ausgangsstoffe, so kann die Umsetzung durch folgendes Reaktionsschema wiedergegeben werden:

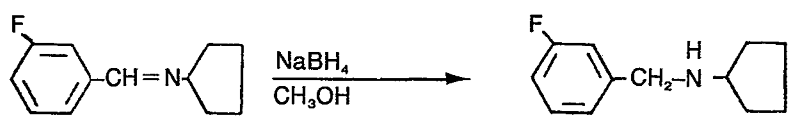


Die bei Herstellung der sekundären Amine der Formel (II) eingesetzten Aldimine der Formel (IV) sind neu. Bei dieser Formel haben die Reste $R^1$ und Ar die Bedeutungen, die bei der Beschreibung der erfindungsgemässen Stoffe der Formel (I) vorzugsweise und besonders bevorzugt für diese Reste genannt wurden. Ihre Herstellung wird weiter unten beschrieben.

Bei der Herstellung der sekundären Amine der Formel (II) werden als Hydrierungsmittel vorzugsweise komplexe Metallhydride, beispielsweise Lithiumalanat, Natriumborhydrid und Cobaltcarbonylwasserstoff, besonders bevorzugt Natriumborhydrid verwendet. Pro Mol Aldimin der Formel (IV) werden etwa 1 Mol Hydrierungsmittel eingesetzt.

Als geeignete Verdünnungsmittel kommen vorzugsweise Alkohole beispielsweise aliphatische Alkohole wie Ethanol, n-Propanol, iso-Propanol, n-Butanol, besonders bevorzugt Methanol in Frage.

Die Umsetzung wird bei einer Temperatur von −20 bis 60 °C, bevorzugt −10 bis 40 °C ausgeführt.

Die Umsetzung wird beispielsweise wie folgt durchgeführt: Das Hydrierungsmittel und der Alkohol werden vorgelegt. Unter Eiskühlung wird dazu das Aldimin der Formel (IV) getropft. Anschliessend wird ca. 1,5 Stunden bei Raumtemperatur gerührt. Nun werden etwa die gleiche Menge Wasser wie eingesetzter Alkohol langsam zugegeben. Anschliessend wird auf übliche Weise mit einem chlorierten Kohlenwasserstoff extrahiert. Die sekundären Amine der Formel (II) können

durch Destillation und/oder Kristallisation isoliert werden.

Verwendet man zur Herstellung der neuen Aldimine der Formel (IV) beispielsweise 3-Fluorbenzaldehyd und Cyclopentylamin, so kann der Reaktionsablauf durch das folgende Formelschema wiedergegeben werden.

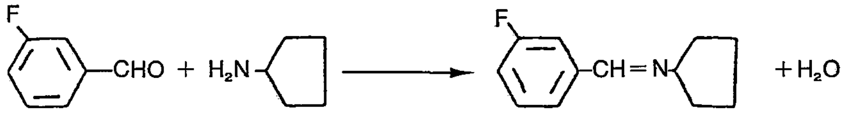

Die bei der Herstellung der Aldimine der Formel (IV) eingesetzten Aldehyde der Formel (V) und Amine der Formel (VI) sind durch die Formeln (V) und (VI) allgemein definiert. Bei diesen Formeln haben die Reste Ar und $R^1$ die Bedeutungen, die bereits im Zusammenhang mit der Beschreibung der erfindungsgemässen Stoffe der Formel (I) vorzugsweise und besonders bevorzugt für diese Reste genannt wurden.

Die eingesetzten Aldehyde der Formel (V) und Amine der Formel (VI) sind allgemein bekannte Verbindungen.

Die Umsetzung der Aldehyde der Formel (V) mit den Aminen der Formel (VI) kann vorzugsweise in einem Verdünnungsmittel durchgeführt werden. Vorzugsweise werden chlorierte Kohlenwasserstoffe, beispielsweise Chloroform und Dichlormethan eingesetzt.

Die Umsetzung wird bei einer Temperatur von 0 bis 100 °C, vorzugsweise bei 20 bis 50 °C, ausgeführt.

Die Umsetzung kann wie folgt ausgeführt werden: Der Aldehyd der Formel (V), gelöst in einem Verdünnungsmittel wird vorgelegt. Dazu wird tropfenweise das Amin der Formel (VI) gegeben. Nach Abklingen der exothermen Reaktion wird etwa 1 Stunde bei Raumtemperatur gerührt. Die Isolierung und Reinigung der Aldimine der Formel (IV) erfolgt auf übliche Art und Weise.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Besonders hervorzuheben ist die gute Wirksamkeit der erfindungsgemässen Wirkstoffe gegen Pellicularia sasakii an Reis. Weiterhin ist eine interessante Wirkung gegen Oomyceten und Schorfpilze zu verzeichnen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen, Trockenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

Beispiel A

Pellicularia-Test (Reis)

Lösungsmittel:

12,5 Gewichtsteile Aceton

Emulgator:

0,3 Gewichtsteile Alkylarylpolyglykolether

Zur Herstellung einer zweckmässigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnass gespritzt. Die Pflanzen verbleiben bis zum Abtrocknen im Gewächshaus. Anschliessend werden die Pflanzen mit Pellicularia sasakii inokuliert und bei 25°C und 100% relativer Luftfeuchtigkeit aufgestellt.

5 bis 8 Tage nach der Inokulation erfolgt die Auswertung des Krankheitsbefalles.

Eine deutliche Überlegenheit in der Wirksamkeit gegenüber dem Stand der Technik zeigen bei diesem Test z.B. die Verbindungen gemäss folgender Herstellungsbeispiele: 9, 13.

Herstellungsbeispiele

Beispiel 1

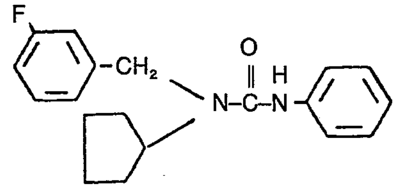

Eine Lösung von 9,65 g (0,05 Mol) N-3-Fluorbenzyl-N-cyclopentylamin in 80 ml wasserfreiem Methylenchlorid wird unter Eiskühlung und Rühren tropfenweise mit 6,1 g (0,05 Mol) Phenylisocyanat versetzt. Man rührt 30 Min. bei Raumtemperatur, 30 Min. bei 40°C, zieht das Lösungsmittel im Vakuum ab und kristallisiert aus Acetonitril um. Es werden 12,8 g N-3-Fluorbenzyl-N-cyclopentyl-N'-phenylharnstoff vom Schmelzpunkt 130°C erhalten.

Beispiel 2

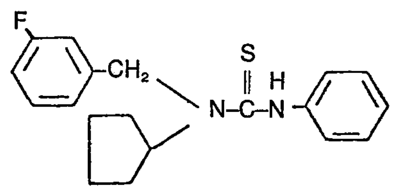

Eine Lösung von 9,65 g (0,05 Mol) N-3-Fluorbenzyl-N-cyclopentylamin in 80 ml Toluol wird rasch tropfenweise mit 6,75 g (0,05 Mol) Phenylisothiocyanat versetzt. Man erhitzt 30 Min. zum Sieden, destilliert das Lösungsmittel im Vakuum ab und kristallisiert aus Isopropanol um. Man erhält 12,5 g N-3-Fluorbenzyl-N-cyclopentyl-N'-phenylthioharnstoff vom Schmelzpunkt 110°C.

Die in Tabelle 3 zusammengestellten Beispiele werden analog hergestellt.

Herstellung der sekundären Amine der Formel (II)

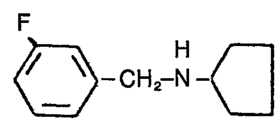

Zu 18,2 g Natriumborhydrid gibt man 160 ml Methanol. Unter Eiskühlung und Rühren tropft man 90,7 g 3-Fluorbenzaldehyd-cyclopentylimid bei 10–15°C zu und rührt anschliessend 1,5 h bei Raumtemperatur. Dann wird mit 120 ml $H_2O$ und 150 ml Methylenchlorid versetzt, die Methylenchlorid-Phase abgetrennt und die wässrige Phase

Tabelle 1

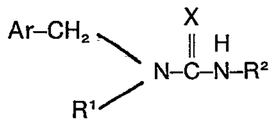

| Bsp. Nr. | X | $R^1$ | $R^2$ | Ar | Schmp. (°C) |
|---|---|---|---|---|---|
| 3 | O | 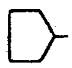 | 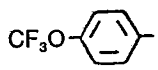 | 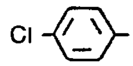 | 139 |
| 4 | O | 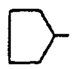 | 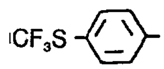 | 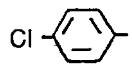 | 147 |
| 5 | O | 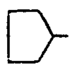 | 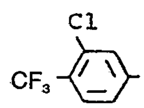 | 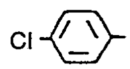 | 167 |
| 6 | O | 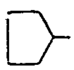 | 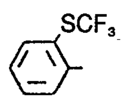 | 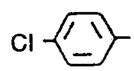 | 90 |
| 7 | O | 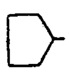 | 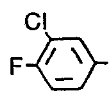 | 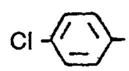 | 132 |
| 8 | O | 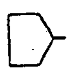 | 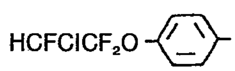 | 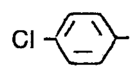 | 128 |
| 9 | O | 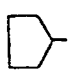 | $C_6H_5$ | 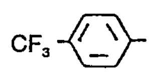 | 133 |
| 10 | O | 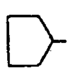 | $C_6H_5$ | 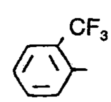 | 191 |
| 11 | O | 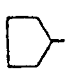 | $C_6H_5$ | 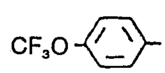 | 108 |
| 12 | O | 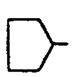 | $C_6H_5$ | 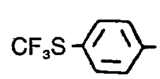 | 149 |
| 13 | O | 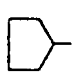 | $C_6H_5$ | 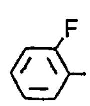 | 153 |
| 14 | O | 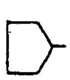 | 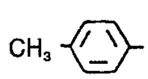 | 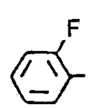 | 149 |

Tabelle 1 (Fortsetzung)

| Bsp. Nr. | X | $R^1$ | $R_2$ | Ar | Schmp. (°C) |
|---|---|---|---|---|---|
| 15 | O | 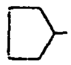 | 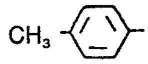 | 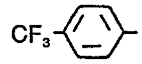 | 141 |
| 16 | O | 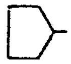 | 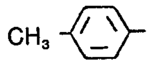 | 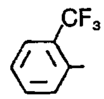 | 198 |
| 17 | O | 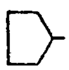 | 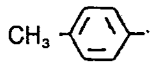 | 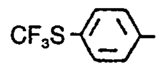 | 149 |
| 18 | O | 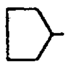 | 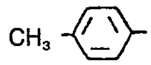 | 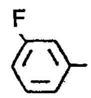 | 145 |
| 19 | O | 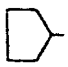 | 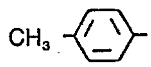 | 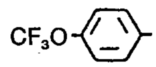 | 125 |
| 20 | O | 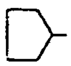 | $CH_3$ | 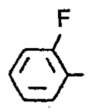 | 145 |
| 21 | O | 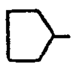 | 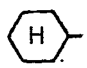 | 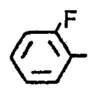 | 109 |
| 22 | O | 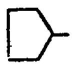 | 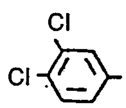 | 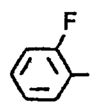 | 144 |
| 23 | O | $CF_3CH_2$– | $C_6H_5$ | $C_6H_5$ | 124 |
| 24 | S | $CF_3CH_2$ | $C_6H_5$ | $C_6H_5$ | 96 |
| 25 | O | $CF_3CH_2$ | 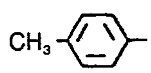 | $C_6H_5$ | 120 |
| 26 | O | $CF_3CH_2$ | $CH_3$ | $C_6H_5$ | 56 |
| 27 | O | $CF_3CH_2$ | 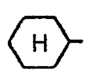 | $C_6H_5$ | 94 |
| 28 | S | 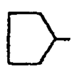 | $C_6H_5$ | 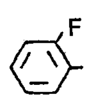 | 81 |

Tabelle 1 (Fortsetzung)

| Bsp. Nr. | X | R¹ | $R_2$ | Ar | Schmp. (°C) |
|---|---|---|---|---|---|
| 29 | S | 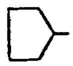 | $C_6H_5$ | 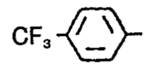 | 129 |
| 30 | S | 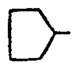 | $C_6H_5$ | 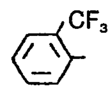 | 138 |
| 31 | S | 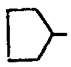 | $C_6H_5$ | 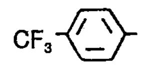 | 119 |
| 32 | S | 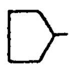 | $C_6H_5$ | 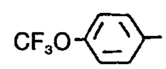 | 127 |

2 mal mit $CH_2Cl_2$ nachextrahiert. Die vereinigten Methylenchlorid-Phasen werden mit Wasser gewaschen, mit $Na_2SO_4$ getrocknet und das Lösungsmittel im Vakuum abgezogen. Die Destillation des Rückstandes in Feinvakuum ergab 82,9 g N-3-Fluorbenzyl-N-cyclopentylamin vom Siedepunkt 10 °C/0,3 bar.

Analog werden die in Tabelle 2 aufgeführten Verbindungen 2 bis 7 hergestellt.

Tabelle 2

$$Ar{-}CH_2{-}\overset{H}{N}{-}R^1$$

| Nr. | Ar | R¹ | Kp (°C/bar) |
|---|---|---|---|
| 2 | 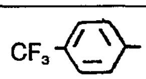 | 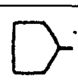 | 119/0,4 |
| 3 | 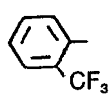 | 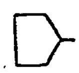 | 112/0,4 |
| 4 | 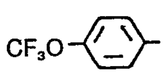 | 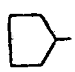 | 107/0,3 |
| 5 | 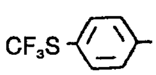 | 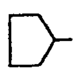 | 132/0,3 |
| 6 | 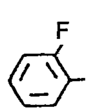 | 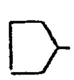 | 103/0,5 |
| 7 | 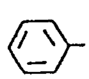 | $CF_3CH_2-$ | 83/22 |

Herstellung der Aldimine der Formel (IV)

Beispiel I

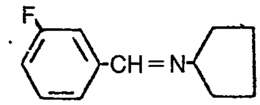

62 g (0,5 Mol) 3-Fluorbenzaldehyd werden in 150 ml Methylenchlorid tropfenweise mit 42,5 g Cyclopentylamin versetzt. Nach dem Abklingen der leicht exothermen Reaktion wird noch 1 h bei Raumtemperatur gerührt, das gebildete Wasser abgetrennt, das Methylenchlorid mit $Na_2SO_4$ getrocknet, filtriert und das Lösungsmittel im Vakuum abdestilliert. Die Destillation des öligen Rückstandes ergab 90,0 g 3-Fluor-benzaldehydcyclopentylimid vom Siedepunkt 80 °C/0,1 bar.

Andere, nach der gleichen Methode hergestellten Verbindungen sind in Tabelle 3 angeführt.

Tabelle 3

| | $Ar-CH=N-R^1$ | | |
|---|---|---|---|
| Nr. | Ar | $R^1$ | Kp (°C/bar) |
| 3.2 | 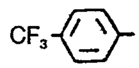 | 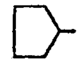 | 104/0,3 |
| 3.3 | 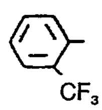 | 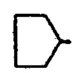 | 95/0,3 |
| 3.4 | 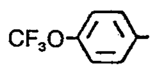 | 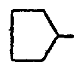 | 104/0,4 |
| 3.5 | 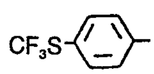 | 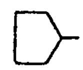 | 133/0,2 |
| 3.6 | 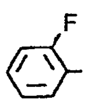 | 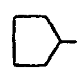 | 121/0,59 |
| 3.7 | 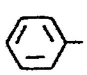 | $CF_3CH_2$ | 43/0,12 |

**Patentansprüche**

1. (Thio-)Harnstoffe der allgemeinen Formel (I)

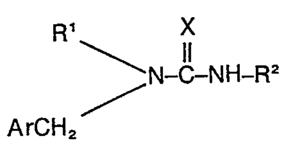

(I)

in welcher

$R^1$ für Cyclopentyl oder 2,2,2-Trifluorethyl steht,

$R^2$ für Methyl, Cyclohexyl oder für gegebenenfalls durch 4-Methyl, 4-Trifluormethoxy, 4-Trifluormethylthio, 2-Trifluormethylthio, 4-Trifluormethylthio-3-chlor, 3-Chlor-4-fluor, 2-Chlor-2-fluor-1,1-difluorethoxy oder 3,4 Dichlor substituiertes Phenyl steht,

Ar für gegebenenfalls durch 4-Chlor, 2-Trifluormethyl, 4-Trifluormethyl, 4-Trifluormethoxy, 4-Trifluormethylthio, 2-Fluor oder 3-Fluor substituiertes Phenyl steht, wobei mindestens einer der Reste $R^1$, $R^2$ und Ar Fluor oder einen fluorhaltigen Substituenten trägt und

X für Sauerstoff oder Schwefel steht.

2. Verfahren zur Herstellung von (Thio-)Harnstoffen der allgemeinen Formel

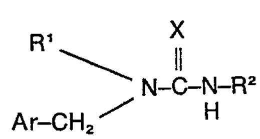

(I),

in welcher

$R^1$ für Cyclopentyl oder 2,2,2-Trifluorethyl steht,

$R^2$ für Methyl, Cyclohexyl oder für gegebenenfalls durch 4-Methyl, 4-Trifluormethoxy, 4-Trifluormethylthio, 2-Trifluormethylthio, 4-Trifluormethylthio-3-chlor, 3-Chlor-4-fluor, 2-Chlor-2-fluor-1,1-difluorethoxy oder 3,4 Dichlor substituiertes Phenyl steht,

Ar für gegebenenfalls durch 4-Chlor, 2-Trifluormethyl, 4-Trifluormethyl, 4-Trifluormethoxy, 4-Trifluormethylthio, 2-Fluor oder 3-Fluor substituiertes Phenyl steht, wobei mindestens einer der Reste $R^1$, $R^2$ und Ar Fluor oder einen fluorhaltigen Substituenten trägt und

X für Sauerstoff oder Schwefel steht,

dadurch gekennzeichnet, dass sekundäre Amine der Formel (II)

$$\underset{\phantom{ArCH_2-}H\phantom{-R^1}}{ArCH_2-N-R^1} \qquad (II)$$

in welcher

Ar und $R^1$ die bei Formel (I) angegebene Bedeutung besitzen

mit (Thio-)Isocyanaten der Formel (III)

$$R^2NCX \qquad (III)$$

in welcher

$R^2$ und X die bei Formel (I) angegebene Bedeutung besitzen,

gegebenenfalls in einem Verdünnungsmittel bei einer Temperatur von −20 bis 150 °C umgesetzt werden.

3. Sekundäre Amine der allgemeinen Formel (II)

$$ArCH_2-NH-R^1 \qquad (II)$$

in welcher

Ar und $R^1$ die in Anspruch 1 angegebene Bedeutung haben.

4. Verfahren zur Herstellung von sekundären Aminen der allgemeinen Formel (II)

$$ArCH_2-NH-R^1 \qquad (II)$$

in welcher

Ar und $R^1$ die in Anspruch 1 angegebene Bedeutung haben,

dadurch gekennzeichnet, dass Aldimine der allgemeinen Formel (IV)

$$ArCH=N-R^1 \qquad (IV)$$

in welcher

Ar und $R^1$ die in Anspruch 1 angegebene Bedeutung haben,

in einem Verdünnungsmittel mit einem Hydrierungsmittel umgesetzt werden.

5. Aldimine der allgemeinen Formel (IV)

$$ArCH=NR^1 \qquad (IV)$$

in welcher

Ar und $R^1$ die in Anspruch 1 angegebene Bedeutung haben.

6. Verfahren zur Herstellung von Aldiminen der allgemeinen Formel (IV)

$$ArCH=NR^1 \qquad (IV)$$

in welcher

Ar und $R^1$ die in Anspruch 1 angegebene Bedeutung haben,

dadurch gekennzeichnet, dass Aldehyde der Formel (V)

$$ArCHO \qquad (V)$$

in welcher

Ar die in Anspruch 1 angegebene Bedeutung hat,

mit primären Aminen der Formel (VI)

$$R^1-NH_2 \qquad (VI)$$

in welcher

$R^1$ die in Anspruch 1 angegebene Bedeutung hat,

gegebenenfalls in einem Verdünnungsmittel bei einer Temperatur von 0 bis 100 °C umgesetzt werden.

7. Fungizide Mittel, gekennzeichnet durch einen Gehalt an mindestens einem (Thio-)Harnstoff der Formel (I).

8. Verfahren zur Bekämpfung von Pilzen, dadurch gekennzeichnet, dass man (Thio-)Harnstoffe der Formel (I) auf Pilze oder ihren Lebensraum einwirken lässt.

9. Verwendung von (Thio-)Harnstoffen der Formel (I) zur Bekämpfung von Pilzen.

**Claims**

1. (Thio)ureas of the general formula (I)

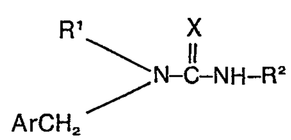

(I)

in which

$R^1$ represents cyclopentyl or 2,2,2-trifluoroethyl,

$R^2$ represents methyl, cyclohexyl or optionally 4-methyl-, 4-trifluoromethoxy-, 4-trifluoromethylthio-, 2-trifluoromethylthio-, 4-trifluoromethylthio-3-chloro-, 3-chloro-4-fluoro-, 2-chloro-2-fluoro-1,1-difluoroethoxy or 3,4 dichloro-substituted phenyl,

Ar represents optionally 4-chloro-, 2-trifluoromethyl-, 4-trifluoromethyl-, 4-trifluoromethoxy-, 4-trifluoromethylthio-, 2-fluoro- or 3-fluoro-substituted phenyl, at least one of the radicals $R^1$, $R^2$ and Ar carrying fluorine or a fluorine-containing substituent, and

X represents oxygen or sulphur.

2. Process for the preparation of (thio)ureas of the general formula

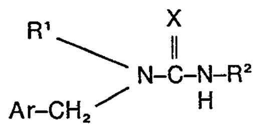 (I),

in which

$R^1$ represents cyclopentyl or 2,2,2-trifluoroethyl,

$R^2$ represents methyl, cyclohexyl or optionally 4-methyl-, 4-trifluoromethoxy-, 4-trifluoromethylthio-, 2-trifluoromethylthio-, 4-trifluoromethylthio-3-chloro-, 3-chloro-4-fluoro-, 2-chloro-2-fluoro-1,1-difluoroethoxy- or 3,4 dichloro-substituted phenyl,

Ar represents optionally 4-chloro-, 2-trifluoromethyl-, 4-trifluoromethyl-, 4-trifluoromethoxy-, 4-trifluoromethylthio-, 2-fluoro- or 3-fluoro-substituted phenyl, at least one of the radicals $R^1$, $R^2$ and Ar carrying fluorine or a fluorine-containing substituent and

X represents oxygen or sulphur,

characterised in that secondary amines of the formula (II)

$$ArCH_2-\underset{H}{N}-R^1 \qquad (II)$$

in which

Ar and $R^1$ have the meaning given under formula (I)

are reacted with (thio)isocyanates of the formula (III)

$$R^2NCX \qquad (III)$$

in which

$R^2$ and X have the meaning given under formula (I),

if appropriate in a diluent, at a temperature of −20 to 150 °C.

3. Secondary amines of the general formula (II)

$$ArCH_2-NH-R^1 \qquad (II)$$

in which

Ar and $R^1$ have the meaning given in Claim 1.

4. Process for the preparation of secondary amines of the general formula (II)

$$ArCH_2-NH-R^1 \qquad (II)$$

in which

Ar and $R^1$ have the meaning given in Claim 1, characterised in that aldimines of the general formula (IV)

$$ArCH=N-R^1 \qquad (IV)$$

in which

Ar and $R^1$ have the meaning given in Claim 1, are reacted with a hydrogenation agent in a diluent.

5. Aldimines of the general formula (IV)

$$ArCH=NR^1 \qquad (IV)$$

in which

Ar and $R^1$ have the meaning given in Claim 1.

6. Process for the preparation of aldimines of the general formula (IV)

$$ArCH=NR^1 \qquad (IV)$$

in which

Ar and $R^1$ have the meaning given in Claim 1, characterised in that aldehydes of the formula (V)

$$ArCHO \qquad (V)$$

in which

Ar has the meaning given in Claim 1,

are reacted with primary amines of the formula (VI)

$$R^1-NH_2 \qquad (VI)$$

in which

$R^1$ has the meaning given in Claim 1,

if appropriate in a diluent at a temperature of 0 to 100 °C.

7. Fungicidal agents, characterised in that they contain at least one (thio)urea of the formula (I).

8. Method of combating fungi, characterised in that (thio)ureas of the formula (I) are allowed to act on fungi or their habitat.

9. Use of (thio)ureas of the formula (I) for combating fungi.

**Revendications**

1. (Thio)-urées de formule générale (I)

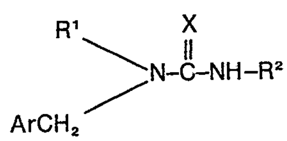 (I)

dans laquelle

$R^1$ est un groupe cyclopentyle ou 2,2,2-trifluoréthyle,

$R^2$ est un groupe méthyle, cyclohexyle, ou un groupe phényle présentant éventuellement une substitution 4-méthyle, 4-trifluorométhoxy, 4-trifluorométhylthio, 2-trifluorométhylthio, 4-trifluorométhylthio-3-chloro, 3-chloro-4-fluoro, 2-chloro-2-fluoro-1,1-difluoréthoxy ou 3,4-dichloro,

Ar désigne un groupe phényle éventuellement substitué par un radical 4-chloro, 2-trifluorométhyle, 4-trifluorométhyle, 4-trifluorométhoxy, 4-trifluorométhylthio, 2-fluoro ou 3-fluoro, l'un au moins des restes $R^1$, $R^2$ et Ar portant du fluor ou un substituant contenant du fluor et

X est l'oxygène ou le soufre.

2. Procédé de production de (thio)-urées de formule générale

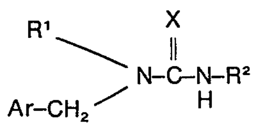 (I),

dans laquelle

$R^1$ est un groupe cyclopentyle ou 2,2,2-trifluoréthyle,

$R^2$ est un groupe méthyle, cyclohexyle, ou un groupe phényle présentant éventuellement une substitution 4-méthyle, 4-trifluorométhoxy, 4-trifluorométhylthio, 2-trifluorométhylthio, 4-trifluorométhylthio-3-chloro, 3-chloro-4-fluoro, 2-chloro-2-fluoro-1,1-difluoréthoxy ou 3,4-dichloro,

Ar désigne un groupe phényle éventuellement substitué par un radical 4-chloro, 2-trifluorométhyle, 4-trifluorométhyle, 4-trifluorométhoxy, 4-trifluorométhylthio, 2-fluoro ou 3-fluoro, l'un au moins des restes $R^1$, $R^2$ et Ar portant du fluor ou un substituant contenant du fluor et

X est l'oxygène ou le soufre,

caractérisé en ce qu'on fait réagir à une température de −20 à 150 °C, éventuellement dans un diluant, des amines secondaires de formule (II)

$$ArCH_2{-}\underset{H}{N}{-}R^1 \qquad (II)$$

dans laquelle

Ar et $R^1$ ont la définition indiquée pour la formule (I)

avec des (thio)-isocyanates de formule (III)

$$R^2NCX \qquad (III)$$

dans laquelle

$R^2$ et X ont la définition indiquée pour la formule (I).

3. Amines secondaires de formule générale (II)

$$ArCH_2{-}NH{-}R^1 \qquad (II)$$

dans laquelle

Ar et $R^1$ ont la définition indiquée dans la revendication 1.

4. Production d'amines secondaires de formule générale (II)

$$ArCH_2{-}NH{-}R^1 \qquad (II)$$

dans laquelle

Ar et $R^1$ ont la définition indiquée dans la revendication 1,

caractérisée en ce qu'on fait réagir avec un agent hydrogénant, dans un diluant, des aldimines de formule générale (IV)

$$ArCH{=}N{-}R^1 \qquad (IV)$$

dans laquelle

Ar et $R^1$ ont la définition indiquée dans la revendication 1.

5. Aldimines de formule générale (IV)

$$ArCH{=}NR^1 \qquad (IV)$$

dans laquelle

Ar et $R^1$ ont la définition indiquée dans la revendication 1.

6. Procédé de production d'aldimines de formule générale (IV)

$$ArCH{=}NR^1 \qquad (IV)$$

dans laquelle

Ar et $R^1$ ont la définition indiquée dans la revendication 1,

caractérisé en ce qu'on fait réagir à une température de 0 à 100 °C, le cas échéant dans un diluant, des aldéhydes de formule (V)

$$ArCHO \qquad (V)$$

dans laquelle

Ar a la définition indiquée dans la revendication 1,

avec des amines primaires de formule (VI)

$$R^1{-}NH_2 \qquad (VI)$$

dans laquelle

$R^1$ a la définition indiquée dans la revendication 1.

7. Compositions fongicides, caractérisées par une teneur en au moins une (thio)-urée de formule (I).

8. Procédé pour combattre des champignons, caractérisé en ce qu'on fait agir des (thio)-urées de formule (I) sur des champignons ou sur leur milieu.

9. Utilisation de (thio)-urées de formule (I) pour combattre des champignons.